# EUROPEAN PATENT APPLICATION

(11) **EP 3 023 051 A1**
(43) Date of publication of application: **25.05.2016**
(21) Application number: 14827076.2
(22) Date of filing: 12.06.2014
(51) Int. Cl.: A61B 5/00

(54) **DIAGNOSTIC APPARATUS USING HABIT, DIAGNOSIS MANAGEMENT APPARATUS, AND DIAGNOSTIC METHOD USING SAME**

(30) Priority: 18.07.2013 KR 20130084994
(71) Applicant: Samsung Electronics Co., Ltd., Suwon-si, Gyeonggi-do 443-742 (KR)
(72) Inventor: LEE, Ho-Sub, Seoul 135-090 (KR)
(74) Representative: Grootscholten, Johannes A.M.
(86) International application number: PCT/KR2014/005162
(87) International publication number: WO 2015/008936

(57) **Abstract**

Disclosed are a diagnostic apparatus for analyzing sensor data detected from one or more sensors to generate user habit data and comparing the generated habit data with data for a diagnosis to diagnose a disease risk group, a diagnosis management apparatus, and a diagnostic method using the same.

## Description

### Technical Field

The following description relates to a technique of collecting behaviors of a user, analyzing the collected behaviors to generate a habit and diagnosing a disease of the user based on the generated habit.

### Background Art

Diseases from lifestyle habits refers to diseases that come from unhealthy or bad habits, and lifestyle habits are highly associated with occurrence of a disease. Thus, a doctor needs to identify a patient's habits through an interview and determine a possibility that a disease may occur or a level of how far a disease has progressed.

Cerebropathia leads to cognitive impairment that causes difficulties for a patient in leading a normal life. A typical disease resulting from lifestyle habit is dementia. As dementia has a lot to do with a patient's lifestyle habits, a doctor may determine a possibility that a patient may develop dementia by objectively observing the patient's habits. In addition, since the symptoms of dementia become worse over time, the doctor may objectively observe the patient's habits to determine if dementia is now progressing, and, if so, perform proper treatments in the early stage of dementia.

As described above, diseases from lifestyle habit are highly associated with a patient's habits, so prevention and treatment of the diseases should start with collecting objective lifestyle habits of the patient. It is only the patient's memory upon which we can depend to identify his or her habits, and thus, it is hard to say that the collected habits are accurate and reliable.

### Technical Problem

The following description relates to providing a diagnostic apparatus and a diagnostic management apparatus to determine whether a user is at risk of a disease based on habit data that are generated through analysis of the user's collected behaviors, and a diagnostic method using the same apparatuses.

### Technical Solution

In one general aspect, there is provided a diagnostic apparatus based on habits, including: a habit analyzer configured to generate a user's habit data by analyzing sensor data detected from at least one sensor; and a diagnoser configured to determine whether the user is at risk of a disease, by comparing the generated habit data with diagnostic data, wherein the diagnostic data is habit data of healthy people.

The habit analyzer may be further configured to comprise a log analyzing module to generate the habit data by analyzing log data stored in a usage log.

The habit analyzer may be further configured to comprise an input analyzing module to generate the habit data by analyzing data input by the user.

The habit analyzer may be further configured to generate the habit data in a normalized form.

The diagnoser may be further configured to comprise a search module to search the diagnostic data that matches profile information of the user.

The diagnoser may be further configured to, in response to a differential value between the habit data and the diagnostic data being greater than a preset threshold, determine that the user is at risk of the disease.

The apparatus may further include a storage configured to store the habit data that are generated at every preset cycle, wherein the diagnoser may be further configured to comprise a tendency analyzing module to determine whether a differential value between the stored habit data and the diagnostic data has a tendency to increase, and, in response to determining that the difference has a tendency to increase, determine that the user is at risk of the disease. Also, the diagnoser may be further configured to comprise a correlation analyzing module to transform a change in a user's habit data stored in the storage into a sequence and analyze correlation between the transformed sequence with a sequence indicating a change in habit data of a patient suffering from a disease, and to, in response to the correlation being greater than a preset threshold, determine that the user is at risk of the disease.

The apparatus may further include a preventive measure provider configured to, in response to a determination that the user is at risk of the disease, provide the user with information on the disease or inform a doctor or a family member of a result of the determination.

In still another general aspect, there is provided a diagnostic management apparatus based on habits, including a habit manager configured to generate a user's habit data by analyzing the user's behavior data received from a diagnostic apparatus; and a diagnosis management configured to determine whether a user is at risk of a disease, by comparing the generated habit data with diagnostic data, wherein the diagnostic data is habit data of healthy people. Here, the behavior data received from the diagnostic apparatus may include at least one of sensor data detected from one or more sensors of the diagnostic apparatus, data directly input by the user through the diagnostic apparatus, and log data stored in a usage log of the diagnostic apparatus.

The apparatus may further include a habit data storage configured to store the habit data that are generated at every preset cycle, wherein the diagnosis manager may be further configured to comprise a tendency analyzing module to determine whether a differential value between habit data stored in the habit data storage and the diagnostic data has a tendency to increase, and, in response to determining that the differential value between the values has a tendency to increase, determine that the user is at risk of the disease. Also, the diagnosis manager may be further configured to comprise a correlation analyzing module to transform a change in habit data stored in the habit data storage into a sequence and analyze the transformed sequence with a sequence indicating a change in habit data of a patient suffering from a disease, and, in response to the correlation being greater than a preset threshold, determine that the user is at risk of the disease.

The apparatus may further include a preventive measure manager configured to provide the user with information on the disease or inform a doctor or a family member of a result of the determination.

In yet another general aspect, there is provided a diagnostic method based on habits, including: searching for diagnostic data that match with profile information of a user, wherein the diagnostic data are habit data of healthy people; and determining whether the user is at risk of a disease, by comparing the user's habit data with the found diagnostic data.

The diagnostic method may further include generating the habit data based on the user's behavior data; wherein the behavior data may include at least one of sensor data detected from one or more sensors, data directly input by the user, and log data stored in a usage log. Also, the generating of the habit data may be normalizing the habit data.

The determining of whether the user is at risk of the disease may include, in response to a differential value between the habit data and the diagnostic data being greater than a preset threshold, determining that the user is at risk of the disease.

The generating of the habit data may include storing the habit data generated at every preset cycle, and the determining of whether the user is at risk of the disease may include determining whether the differential value between the stored habit data and the diagnostic data has a tendency to increase, and, in response to a determination that the differential value between the values has a tendency to increase, determining that the user is at risk of the disease. Also, the determining of whether the user is at risk of the disease comprises transforming a change in the stored habit data into a sequence, analyzing correlation between the transformed sequence with a sequence that indicates a change in habit data of a patient suffering from a disease, and, in response to the correlation being greater than a preset threshold, determining that the user is at risk of the disease.

The diagnostic method may further include, in response to a determination that the user is at risk of the disease, either providing the user with information on the disease or informing a doctor or a family member of a result of the determination.

### Advantageous Effects

A user's behaviors are observed so as to analyze the user's habit, and based on the analyzed habit, whether the user is at a risk of a disease is determined in advance. In addition, the user's habit is objectively analyzed by using a diagnostic apparatus, so that an accuracy of the diagnosis of whether a user is at risk of a disease may be improved.Moreover, whether a user is at a risk of a disease is determined through a comparison between diagnostic data that matches a user's profile information, and the user's habit data, thereby reducing the errors in determination as to whether the user is at a risk of a disease.

Furthermore, in response to a determination that the user is at a risk of the disease, various preventive measures are provided so that the initial reaction to the disease may be performed. Also, the habit data may be used as reference data for disease treatment and symptom relief.

### Description of Drawings

FIG. 1 is a block diagram illustrating an example of a diagnostic system based on habits.
FIG. 2 is a block diagram illustrating a diagnostic apparatus based on habits according to an exemplary embodiment.
FIG. 3 is a block diagram illustrating a sensor according to an exemplary embodiment.
FIG. 4 is a block diagram illustrating a habit analyzer according to an exemplary embodiment.
FIG. 5 is a block diagram illustrating a diagnoser according to an exemplary embodiment.
FIG. 6 is a block diagram illustrating a diagnostic management apparatus according to another exemplary embodiment.
FIG. 7 is a block diagram illustrating a habit manager according to another exemplary embodiment.
FIG. 8 is a block diagram illustrating a diagnosis manager according to another exemplary embodiment.
FIG. 9 is a flow chart illustrating a diagnostic method based on habits according to an exemplary embodiment.
FIG. 10 is a view for explaining a method for generating habit data according to an exemplary embodiment.
FIG. 11 is a flow chart illustrating an example of operation 103 shown in FIG. 9.
FIG. 12 is a diagram for explaining an example of operation 103 shown in FIG. 9.
FIG. 13 is a flow chart illustrating another example of operation 103 shown in FIG. 9.
FIG. 14 is a diagram for explaining another example of operation 103 shown in FIG. 9.
FIG. 15 is a diagram for explaining another example of operation 103 shown in FIG. 9.

### Mode for Invention

The following description is provided to assist the reader in gaining a comprehensive understanding of the methods, apparatuses, and/or systems described herein. Accordingly, various changes, modifications, and equivalents of the methods, apparatuses, and/or systems described herein will be suggested to those of healthy skill in the art. Also, descriptions of well-known functions and constructions may be omitted for increased clarity and conciseness.

Terms used throughout the following description are selected in consideration of functions thereof in exemplary embodiments. The meaning of the terms may vary according to a user, intention of an operator or practices. Thus, if specific definition of a term is provided, the term is understood as the definition goes. However, if not, the term may be understood by a general sense of those skilled in the art.

In addition, although the aspects or configurations of exemplary embodiments throughout the following description are provided as one combined configuration in accompanying drawings, it needs to be understood that they may not be combined or may be combined freely, if those skilled in the art consider them as technical contradictions.

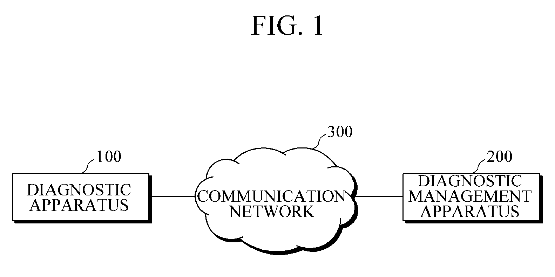

FIG. 1 is a block diagram illustrating an example of a diagnostic system based on habits.

The diagnostic system shown in FIG. 1 collects a user's behaviors using various apparatuses 100 for diagnosis to analyze the user's habit, and determines whether the user is at risk of a disease based on the analysis result. In addition, if it is determined that the user is at risk of the disease, the diagnostic system may perform various preventive measures to prevent the disease. The concept of being at risk of a disease embrace broad meaning, including not only the fact that a user is highly likely to develop the disease, but also the fact that the user has symptoms of a specific early-stage disease. The disease may be developed due to bad lifestyle habits, and includes diseases that are highly associated with the user's habits. For example, the disease includes all onset of diseases that highly associated with habits, such as dementia, diabetes, high blood pressure and other adult diseases.

Referring to FIG. 1, the diagnostic system includes a diagnostic apparatus 100 and a diagnostic management apparatus 200.

The diagnostic apparatus 100 collects a user's behaviors in various ways, and analyze the user's habit based on the collected behaviors. A habit refers to a behavior pattern that the user repeats in daily life. In addition, the diagnostic apparatus 100 determines that the user is at risk of a disease based on the analyzed habit, and, in response to a determination that the user is at risk of the disease, provides preventive measures. The diagnostic apparatus 100 may request and receive data necessary for such determination and preventive measure from the diagnostic management apparatus 200, and, if necessary, may transmit the data to the diagnostic management apparatus 200.

In one embodiment, in response to a request from the diagnostic apparatus 100, the diagnostic management apparatus 200 may provide data the diagnostic apparatus 100. In addition, the diagnostic management apparatus 200 may receive various kinds of data from the diagnostic apparatus 100 for management. Herein, the diagnostic management apparatus 200 may include a cloud computing device.

In another embodiment, in response to a request from the diagnostic apparatus 100, the diagnostic management apparatus 200 may perform some of the operations assigned to the diagnostic apparatus 100 and may transmit the operation results to the diagnostic apparatus 100. For example, the diagnostic management apparatus 200 may receive collected behaviors, analyze a user's habit based on the collected behaviors, and determine whether the user is at risk of a disease. In addition, a diagnosis manager 230 included in the diagnostic management apparatus 200 may perform appropriate preventive measures when the user is determined to be at risk of a disease.

A communication network 300 may include various types of communication networks. For example, the communication network 300 may include an Internet Protocol (IP)-based network which is capable of transmitting and receive massive data, an All-IP network where different networks are combined based on IP, a wireless local area network (LAN), such as Wireless Broadband (Wibro) and Wi-Fi, a Wireless Personal Area Network (WPAN), a mobile communication network, a wired communication network, and a satellite communication network. In addition, it needs to be understood that the communication network 300 includes not just other well-known communication networks, but also all the communication networks that will be developed in the future.

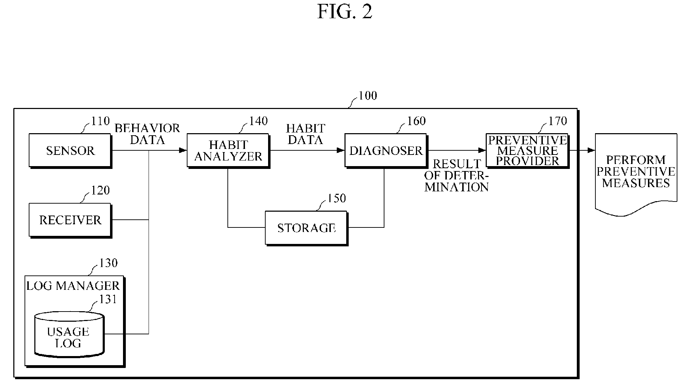

FIG. 2 is a block diagram illustrating a diagnostic apparatus based on habits according to an exemplary embodiment.

A diagnostic apparatus 100, shown in FIG. 2, may be a device that is comfortable to carry around, including a mobile communication terminal, a smart phone, a Portable Medial Player (PMP), a Personal Digital Assistant (PDA), and a Tablet PC. Although the diagnostic apparatus 100 is not limited thereto, a device with various sensors, such as a smart phone, is useful in collecting behavior data using hardware and software of the smart phone to generate habit data based on the collected behavior data.

Referring to FIG. 2, the diagnostic apparatus 100 based on habits includes a sensor 110, a receiver 120, a log manager 130, a habit analyzer 140, a storage 150, a diagnoser 160 and a preventive measure provider 170. The diagnostic apparatus 100 in FIG. 2 may collect a user's behavior data, analyze the collected habit data to generate habit data, and determine whether the user is at risk of a disease based on the generated habit data. In addition, in response to a determination that the user is at risk of the disease, the diagnostic apparatus 100 may provide preventive measures for the disease.

Behavior data is data of behaviors that are collected by the diagnostic apparatus 100 from a user's daily life, including movements, exercising, sleeping, eating and emotions. For example, behavior data may be sensor data detected by the sensor 110, data input by a user, or log data stored in a usage log 131.

Habit data is data of a user's habit, which is generated through analysis of behavior data. A piece of habit data may include a plurality of habit factors. For example, a piece of habit data may include factors relating to an eating habit, a work-out habit, and an emotional habit. In addition, one habit factor of habit data may be generated by analyzing a plurality of pieces of behavior data. For example, a work-out habit may be generated by analyzing a plurality of pieces of behavior data (that is, input data and data detected by a sensor). It needs to be understood that habit data may have different habit factors according to a disease or a diagnostic apparatus.

The sensor 110 generates sensor data. More specifically, the sensor 110 detects external information or a change of external information of the diagnostic apparatus 100, and generates sensor data based on the detected external information or the detected change in external information.

The receiver 120 may receive data from a user. The receiver 120 may include various kinds of input means, such as a key pad, a touch screen, a camera and a microphone. Accordingly, a user may input data using voice or may input an image captured by a camera as data. For example, the receiver 130 may receive voice data from a user or image-type data of a user's facial expression captured by a camera. In addition, the receiver 120 may receive the user's response for a preset query in order to collect the user's behaviors. At this point, the receiver 120 may receive the user's voice or an image as the response.

The log manager 130 manages log data. More specifically, the log manager 130 may store monitored log data. The log manager 130 may further include the usage log 131 to store log data. The log data refers to information about an event that occurs in the diagnostic apparatus 100 over time. For example, in the case where the diagnostic apparatus 100 is a smart phone, the log data is information about various events that occur in the smart phone, including phone call history, transmitted/received text message history, internet search history, and application usage history.

The habit analyzer 140 generates habit data of a user by analyzing the user's behavior data. More specifically, the habit analyzer 140 may generate habit data of a user by analyzing the user's repeated habits based on habit data, such as sensor data detected by the sensor 110, data input from the user through the receiver, and log data stored in the usage log 131. The habit analyzer 140 may analyze one or more pieces of habit data to generate habit data having one or more habit factors. For example, in the case where a piece of habit data covers a eating habit, a work-out habit, and an emotional-state, the habit analyzer 140 may generate a eating habit factor, an work-out habit factor and an emotional-state factor by analyzing eating-habit related behavior data input by the user, sensor data detected through an accelerator and the user's voice data input through phone calls. Alternatively, the habit analyzer 140 may generate a single factor for habit data by analyzing a plurality of pieces of behavior data..

The storage 150 stores and manages various types of data that are necessary to operate the diagnostic apparatus 100. More specifically, the storage 150 may store habit data that are generated at every preset cycle. In addition, the storage 150 may store behavior data. Furthermore, the storage 150 may store a sequence that indicates a change in each factor of diagnostic data or a change in each factor of a patient's habit data. Furthermore, the storage 150 may store data that are necessary to provide various preventive measures in order to prevent a disease.

The diagnoser 160 determines whether a user is at risk of a disease based on the user's habit data. More specifically, the diagnoser 160 may determine whether a user is at risk of a disease by comparing the user's habit data with diagnostic data. The diagnostic data refers to reference habit data that are used as a standard when determining whether a user is at risk of a disease. The diagnostic data may be habits of healthy people. In addition, the diagnostic data may differ according to a user's profile information (e.g., gender, age, race and nationality). The diagnostic data may be stored in the storage 150 of the diagnostic apparatus 100 or may be received from the diagnostic management apparatus 200 (referring to FIG. 1). In addition, the diagnoser 160 may analyze correlation between a sequence indicating a change in a user's habit data with a sequence indicating a change in a patient's habit data, and then may determine whether the user is at risk of a disease.

The preventive measure provider 170 provides various preventive measures to prevent a disease. The preventive measure provider 170 may provide a user with various kinds of information including a criterion necessary for determining whether a user is at risk of a disease, a cause of the disease, preventive measures to avoid the disease, and measures to mitigate the progress of the disease. The information on a disease may be stored in the storage 150 or may be provided from the diagnostic management apparatus 200 (referring to FIG. 1).

In addition, the preventive measure provider 170 may inform a doctor or a user's family member of a result of determination as to whether the user is at risk of a disease. To this end, the storage 150 may store information about the doctor or the user's family member. In addition, the preventive measure provider 170 may provide the doctor with habit data generated by the habit analyzer 140. Furthermore, through the diagnostic management apparatus 200 (referring to FIG. 1), the preventive measure provider 170 may inform the doctor or the user's family of a result of determination whether the user is at risk of a disease.

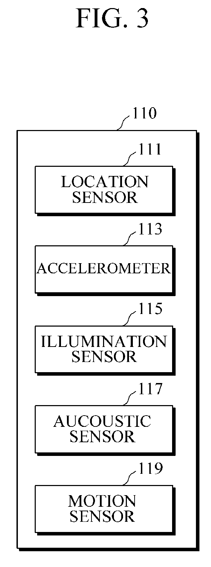

FIG. 3 is a block diagram illustrating a sensor according to an exemplary embodiment.

The sensor 110, shown in FIG. 2, detects external information or a change in external information of a diagnostic apparatus to generate sensor data. The sensors shown in FIG. 3 may be included in sensors of a diagnostic apparatus, but sensors of the diagnostic apparatus are not limited thereto.

Referring to FIG. 3, the sensor 110 includes a position sensor 111, an accelerometer 130, an illumination sensor 115, an acoustic sensor 117 and a motion sensor 119.

The position sensor 111 generates sensor data by detecting a position of a diagnostic apparatus. More specifically, the position sensor 111 may detect a position of a diagnostic apparatus, and generate sensor data relative to the apparatus's change in environment based on the position detected for a predetermined period of time. Meanwhile, the position sensor 111 may be a global positioning system (GPS).

The accelerometer 113 generates sensor data by detecting a change in acceleration of a diagnostic apparatus. More specifically, the accelerometer 113 may detect a change in acceleration, vibration and impact of a diagnostic apparatus, and may generate acceleration-related sensor data based on the detected change.

The illumination sensor 115 generates sensor data by detecting brightness in the surroundings of a diagnostic apparatus. More specifically, the illumination sensor 115 may detect brightness in the surroundings of a diagnostic apparatus and generate brightness-related sensor data based on the detected brightness.

The acoustic sensor 117 generates sensor data by detecting sound in the surrounding of a diagnostic apparatus. More specifically, the acoustic sensor 117 may detect sound in the surroundings of a diagnostic apparatus and generate noise-related sensor data based on the detected sound. In addition, the acoustic sensor 117 may detect a user's phone call voice and generate sensor data based on the detected phone call voice. In this case, the generated sensor data may be used to generate emotional-state-related habit data through machine learning.

The motion sensor 119 generates sensor data by detecting movements in the surroundings of a diagnostic apparatus. More specifically, the motion sensor 119 may detect movements in the surroundings of the diagnostic apparatus and generate sensor data based on the detected motions.

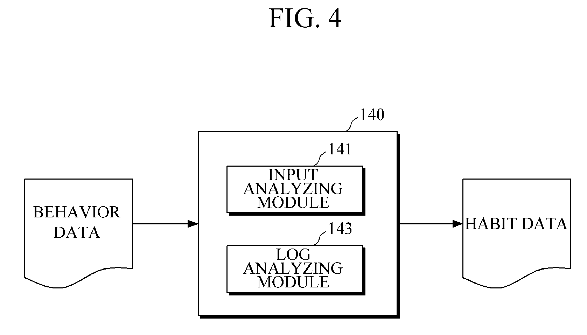

FIG. 4 is a block diagram illustrating a habit analyzer according to an exemplary embodiment.

Referring to FIG. 4, a habit analyzer 140 includes an input analyzing module 141 and a log analyzing module 143. The habit analyzer 140 generates habit factors for habit data by analyzing habits of a user based on behavior data, such as sensor data detected from a sensor, data directly input by the user and log data stored in a usage log. The habit analyzer 140 may analyze behavior data using a machine learning technique. The machine learning technique is a technique of analyzing new data based on known properties learned from training data. The habit data generated by the habit analyzer 140 may be data that has been normalized. Normalization is a process of altering habit data based on environmental differences between the habit data and diagnostic data to perform comparison therebetween. For example, each habit factor for habit data may be normalized to a value ranged from 0 to 1. Normalization may make it easy to compare habit data with diagnostic data and to manage the habit data. In addition, the habit analyzer 140 may store habit data generated at every preset cycle.

Moreover, the habit analyzer 140 may further include the input analyzing module 141. The input analyzing module 141 generates habit data by analyzing data input by a user. Specifically, the input analyzing module 141 may generate an emotion-related habit factor for habit data by analyzing voice input through a microphone and image data input through a camera. In this case, the input data may be the user's response for a query that is predetermined to collect a behavior of the user.

Furthermore, the habit analyzer 140 may further include the log analyzing module 143. The log analyzing module 143 generates habit data by analyzing log data stored in a usage log. Specifically, the log analyzing module 143 may generate habit data by analyzing log data, such as call history, transmitted/received text message history, internet search history and application usage history.

Hereinafter, an example in which a habit analyzer generates habit data is described with reference to FIGS. 3 and 4.

The habit analyzer 140 may generate a movement-related habit factor for habit data by analyzing a distance travelled by a user during a day based on sensor data regarding the user's locations detected from the location sensor 111.

The habit analyzer 140 may generate a work-out-related habit factor for habit data by analyzing sensor data regarding a change in acceleration, which is detected from an accelerometer 113. Specifically, the habit analyzer 140 may analyze the sensor data through machine learning, classify acceleration data into specific states (walking, running or stopping), and generate a work-out-related habit factor for habit data based on a specific classified state.

The habit analyzer 140 may generate sleeping-related habit data by analyzing sensor data regarding brightness detected from an illumination sensor 115. The habit analyzer 140 may analyze the sensor data through machine learning, classify illumination data into specific states (before sleep, during sleep or after waking), and generate a sleeping-related habit factor for habit data based on a specific classified state. In addition, the habit analyzer 140 may generate a sleep-quality-related habit factor for habit data by analyzing both sensor data regarding motions detected from a motion sensor 119 and sensor data regarding sound detected from an acoustic sensor 117. In another example, the habit analyzer 140 may generate only one factor for habit data by analyzing multiple pieces of habit data.

In addition, the input analyzing module 141 may generate a user's eating habit-related habit factor for habit data by analyzing data regarding the user's food intake per meal, quantity of smoking and alcohol intake, which are input by the user in response to a predetermined query. Furthermore, the input analyzing module 141 may generate a user's eating habit-related habit factor for habit data by analyzing food images captured by the user using a camera.

The log analyzing module 143 may collect a user's emotion-related habit factor for habit data by analyzing the user's text message history using a machine learning technique, or may collect a user's social life-related habit factor for habit data by analyzing the user's call history. In addition, the log analyzing module 143 may generate a user's recognition activity-related habit factor for habit data by analyzing the user's internet search history or application usage history. Furthermore, the log analyzing module 143 may generate a user's habit data by analyzing payment information included in the user's text messages.

However, the above-described examples are provided to describe in detail how the habit analyzer 140 generates habit data, but aspects of the present disclosure are not limited thereto.

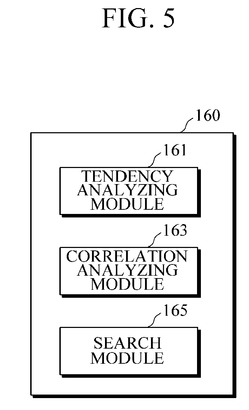

FIG. 5 is a block diagram illustrating a diagnoser according to an exemplary embodiment.

Referring to FIG. 5, a diagnoser 160 determines whether a user is at risk of a disease, by comparing the user's habit data with pre-stored diagnostic data. More specifically, the diagnoser 160 may calculate a sum of differences between respective factor for habit data and corresponding factor for diagnostic data, and, in response to the sum being greater than a preset threshold, determine that the user is at risk of a disease. However, a habit factor with a value of NULL may not be taken into account for the calculation. In addition, in the case where habit data is generated at every preset cycle, the diagnoser 160 may determine whether a user is at risk of a disease, by using an arithmetic mean among the plurality pieces of habit data.

The diagnoser 160 may further include a tendency analyzing module 161. The tendency analyzing module 161 may determine whether a differential value between habit data and diagnostic data has a tendency to increase. More specifically, the tendency analyzing module 161 determines whether a sum of differences between respective factors for habit data and corresponding factors for diagnostic data has a tendency to increase. In response to a determination made by the tendency analyzing module 161 that the sum has a tendency to increase, the diagnoser 160 may determine that the user is at risk of a disease.

The diagnoser 160 may further include a correlation analyzing module 163. The correlation analyzing module 163 analyzes correlation between each respective habit factor for habit data of a user and each corresponding habit factor for habit data of a patient suffering from a disease. In response to the correlation being greater than a preset threshold, the diagnoser 160 may determine that the user is at risk of the disease. In order to analyze the correlation, the correlation analyzing module 163 may transform a change in each factor of a plurality of pieces of habit data into a sequence, and compare the transformed sequence with a sequence that indicates a change in each factor for the habit data of the patient. At this point, in order to analyze the correlation, the correlation analyzing module 163 may perform regression analysis on the user's habit data and the patient's habit data.

The diagnoser 160 may further include a search module 165. The search module 165 detects a user's profile information and searches for diagnostic data that matches the detected profile information. For example, the search module 165 detects profile information, for example, age, gender, race and nationality, of a user of a diagnostic apparatus, and searches for diagnostic data that matches the detected profile information.

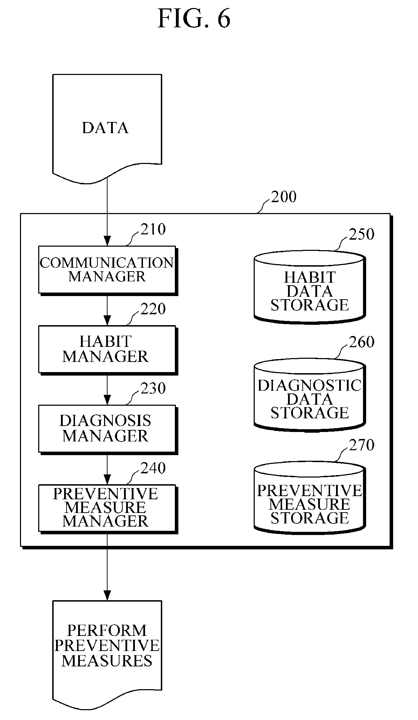

FIG. 6 is a block diagram illustrating a diagnostic management apparatus according to another exemplary embodiment.

Referring to FIG. 6, a diagnostic management apparatus 200 includes a communication manager 210, a habit manager 220, a diagnosis manager 230 and a preventive measure manager 240. The diagnostic management apparatus 200 shown in FIG. 6 generate habit data by analyzing behavior data received from a diagnostic apparatus. The diagnostic management apparatus 200 may determine whether a user is at risk of a disease based on the user's habit data, and, in the case where the user is at risk of the disease, perform proper preventive measures.

The communication manager 210 transmits and receives data over a communication network. The communication manager 210 may receive behavior data from a diagnostic apparatus, which is connected over a communication network. In addition, the communication manager 210 may transmit various types of data to the diagnostic apparatus over the communication network.

The habit manager 220 generates a user's habit data by analyzing the user's behavior data received through the communication manager 210. Specifically, the habit manager 220 may generate habit factors for habit data by analyzing the user's repetitive behaviors based on behavior data, such as sensor data detected from a sensor, data input directly from the user and log data stored in a usage log. Meanwhile, the habit manager 220 may store generated habit data in a habit data storage.

The diagnosis manager 230 may determine whether a user is at risk of a disease based on habit data. More specifically, in response to a determination that a differential value between diagnostic data and habit data is greater than a preset threshold, the diagnosis manager 230 may determine that a user is at risk of a disease. Herein, diagnostic data, which is reference habit data used to determine whether a user has developed a disease, may be habit data of healthy people. Such diagnostic data may be stored in the diagnostic data storage 260.

The preventive measure manager 240 performs various preventive measures to prevent a disease. The preventive measure manager 240 may provide a user with information on various diseases, which includes a discriminant criteria as to whether a user is at risk of a disease, preventive measures to prevent the disease and measures of mitigating the process of the disease. In addition, the preventive measure manager 240 may inform a pre-registered family member or a doctor of a result of the determination as to whether a user is at risk of a disease.

The habit data storage 250 stores habit data. Herein, habit data refers to a user's habit-related data that are generated through analysis of the user's behavior data. A piece of habit data may include a plurality of habit factors. A habit factor for habit data may be generated by analyzing a plurality pieces of behavior data. In addition, a habit data storage 250 may sequentially store habit data that are generated at every preset cycle.

The diagnostic data storage 260 stores diagnostic data. Herein, diagnostic data refers to reference habit data used as a criteria to determine whether a user is at risk of a disease. The diagnostic data storage 260 may store a plurality of pieces of diagnostic data that are generated based on a user's profile information. For example, the diagnostic data storage 260 may store a plurality of pieces of diagnostic data that are generated based on gender, age, race and nationality of a user. In another example, the diagnostic data may be habit data of healthy people. In addition, the diagnostic data storage 260 may further include a sequence indicating a change in each factor for habit data of a patient suffering from a disease.

The preventive measure storage 270 stores information about various preventive measures to prevent a disease. More specifically, the preventive measure storage 270 may include information on a disease, and may store various kinds of information to inform a doctor or a family member that a user is at risk of a disease.

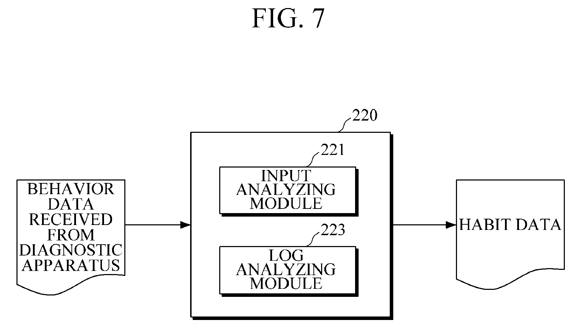

FIG. 7 is a block diagram illustrating a habit manager according to another exemplary embodiment.

Referring to FIG. 7, a habit manager 220 include an input analyzing module 221 and a log analyzing module 223. The habit manager 220 generate each habit factor for habit data by analyzing a user's habit based on behavior data received from a diagnostic data. The habit manager 220 may analyze the behavior data using a learning machine technique. At this time, the habit data may be generated in a normalized form.

The habit manager 220 may generate habit data by analyzing sensor data received from a diagnostic apparatus, the sensor data which is detected from one or more sensors. The sensor data detected from a sensor may differ according to types of sensors provided in the diagnostic apparatus.

The habit manager 220 may further include an input analyzing module 221. The input analyzing module 221 generates a user's habit data by analyzing data input from the user. Specifically, the input analyzing module 221 may generate a user's emotion-related habit factor for habit data by generating voice data input through a microphone and image data input through a camera.

The habit manager 220 may further include a log analyzing module 223. The log analyzing module 223 generates habit data by analyzing log data stored in a usage log. Specifically, the log analyzing module 223 may generate habit data by analyzing log data, such as call history, transmitted/received text message history, internet search history and application usage history.

In another example, the habit manager 220 may generate only one habit factor for habit data by analyzing a plurality of pieces of behavior data. In addition, the habit analyzer 220 may store habit data in a storage by analyzing behavior data at every preset cycle.

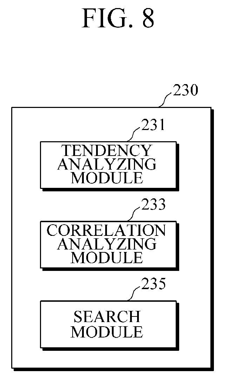

FIG. 8 is a block diagram illustrating a diagnosis manager according to another exemplary embodiment.

Referring to FIG. 8, the diagnosis manager 230 determines whether a user is at risk of a disease, by comparing the user's habit data with pre-stored diagnostic data. Specifically, the diagnosis manager 230 may calculate a sum of differences between respective habit factors for habit data and corresponding factors for diagnostic data. In response to the sum being greater than a preset threshold, the diagnosis manager 230 may determine that the user is at risk of a disease. Meanwhile, the diagnosis manager 230 may not take a factor with a value of NULL into account for the calculation. In addition, in the case where a plurality of pieces of habit data are generated at every preset cycle, the diagnosis manager 230 may determine whether a user is at risk of a disease, by using an arithmetic mean among the plurality pieces of habit data.

The diagnosis manager 230 may further include a tendency analyzing module 231. The tendency analyzing module 231 may determine whether a differential value between habit data and diagnostic data has a tendency to increase. Specifically, the tendency analyzing module 231 determines whether a sum of differences between respective habit factors for habit data and corresponding habit factors for diagnostic data has a tendency to increase. In response to a determination that the sum has a tendency to increase, the diagnosis manager 230 may determine that the user is at risk of a disease.

The diagnosis manager 230 may further include a correlation analyzing module 233. The correlation analyzing module 233 analyzes correlation between each respective habit factor for habit data of a user and each corresponding habit for habit data of a patient suffering from a disease. In response to the correlation being greater than a preset threshold, the diagnosis manager 230 may determine that the user is at risk of the disease. In order to analyze the correlation, the correlation analyzing module 233 may transform a change in each factor for a plurality of pieces of habit data into a sequence, and compare the transformed sequence with a sequence that indicates a change in each factor for habit data of the patient. At this point, in order to analyze the correlation, the correlation analyzing module 233 may perform regression analysis on the user's habit data and the patient's habit data.

The diagnosis manager 230 may further include a search module 235. The search module 235 detects a user's profile information, and searches for diagnostic data that matches the detected profile information. For example, the search module 235 may detect profile information, such as age, gender, race and nationality, of a user of a diagnostic apparatus, and searches a diagnostic data storage for diagnostic data that matches the detected profile information.

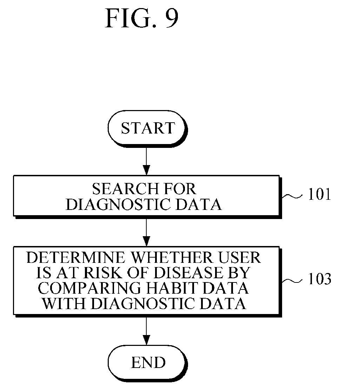

FIG. 9 is a flow chart illustrating a diagnostic method based on habits according to an exemplary embodiment.

Referring to FIG. 9, a diagnostic apparatus searches for diagnostic data that matches profile information of a user in operation 101. At this point, the diagnostic apparatus may extract the profile information in order to search for the diagnostic data. In addition, the diagnostic apparatus may request a diagnostic management apparatus to search for the diagnostic data which matches the profile information, and receives the diagnostic data from the diagnostic management apparatus. Herein, diagnostic data may be habit data of healthy people. In addition, diagnostic data may differ according to a user's profile information, such as gender, age, race and nationality.

In operation 103, the diagnostic apparatus determines whether the user is at risk of a disease, by comparing habit data with the diagnostic data.

Specifically, in operation 103, the diagnostic apparatus determines whether a user is at risk of a disease, by comparing habit data of the user with diagnostic data. More specifically, the diagnostic apparatus calculates a differential value between the habit data and the diagnostic data, and, in response to the calculated differential value being greater than a preset threshold, determines that the user is at risk of the disease. The preset threshold may be changed according to setting information.

The method illustrated in FIG. 9 is described with reference to a diagnostic apparatus, but the same method may be used by a diagnostic management apparatus to perform diagnosis of a disease.

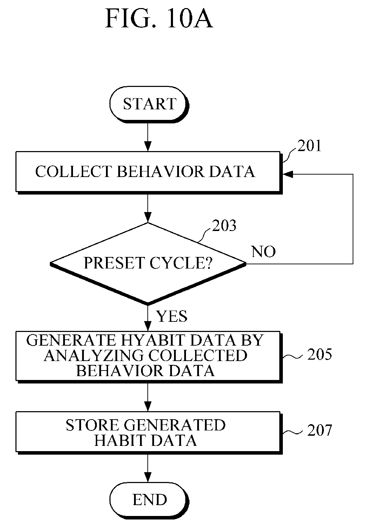

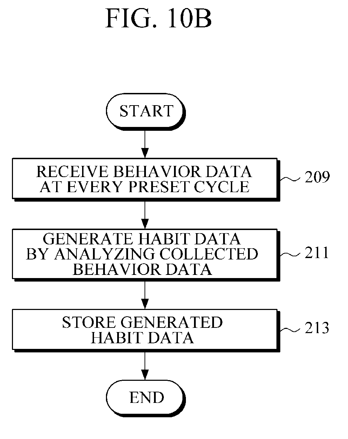

FIG. 10 is a flow chart illustrating a method for generating habit data according to an exemplary embodiment.

FIG. 10A is an example of a method whereby a diagnostic apparatus generates habit data.

Referring to FIG. 10A, a diagnostic apparatus collects behavior data in operation 201. At this point, the diagnostic apparatus may collect behavior data in various ways. Specifically, the diagnostic apparatus may receive sensor data detected from one or more sensors, behavior data directly input by a user and log data regarding the user's application usage history. In order to collect behavior data, the diagnostic apparatus may periodically detect a change in a sensor and/or may request the user to input a response for a predetermined query. In addition, in the case of performing a different function, the diagnostic apparatus may generate log data by monitoring the user's usage history of the different function. For example, when the diagnostic apparatus is a smart phone, the diagnostic apparatus may collect the user's smart phone usage history, such as call history, transmitted/received text message history, internet search history and application usage history, in the form of log data.

In operation 203, the diagnostic apparatus determines whether a preset cycle has passed. In the case where the preset cycle has passed, the diagnostic apparatus goes back to operation 201 to collect behavior data. For example, in the case where behavior data is set to be generated at noon every day, the diagnostic apparatus collects behavior data again if the time has not reached noon.

In operation 203, in response to a determination that the preset cycle has passed, the diagnostic apparatus moves on into operation 205 to analyze collected behavior data to thereby generate habit data. Specifically, the diagnostic apparatus generates each habit factor by analyzing a user's habit based on the collected behavior data. The diagnostic apparatus may analyze the behavior data using a machine learning technique, and the habit data may be generated in a normalized form. Herein, habit data refers to a user's habit-related data that are generated through analysis of the user's behavior data. A piece of habit data may include a plurality of habit factors. Each habit factor for habit data may be generated by analyzing a plurality of pieces of behavior data.

The habit data generated in operation 205 is stored in operation 207. In this case, the habit data may be stored either in the diagnostic apparatus or in the diagnostic management apparatus.

FIG. 10B is an example of a method whereby a diagnostic apparatus generates habit data. Referring to FIG. 10B, a diagnostic apparatus receives behavior data from a diagnostic management apparatus at each preset cycle. For example, the diagnostic management apparatus may receive behavior data every 24 hours.

The diagnostic management apparatus generates habit data by analyzing the received behavior data in operation 211, and stores the generated habit data in operation 213.

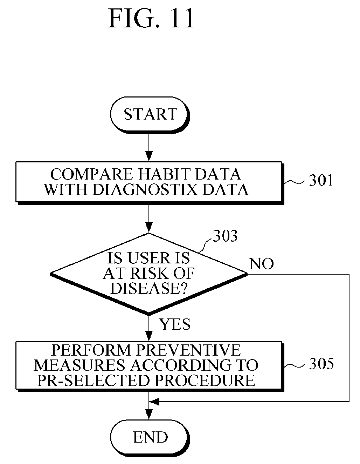

FIG. 11 is a flow chart illustrating an example of operation 103 shown in FIG. 9.

Referring to FIG. 11, a diagnostic apparatus compares a user's habit data with diagnostic data in 301. Specifically, the diagnostic apparatus may calculate differences between respective factor for the user's habit data and corresponding factors of the diagnostic data, and calculate a sum of the differences. At this point, the diagnostic apparatus may not take a habit factor with a value of null into account for the calculation. In addition, in the case of comparing a plurality of pieces of habit data with a single piece of diagnostic data, the diagnostic apparatus may calculate a sum of the differences between respective habit factors for the user's habit data and corresponding factors of the diagnostic data, by using an arithmetic mean of differences among the plurality pieces of habit data. For example, the diagnostic apparatus may calculate a mean value of each habit factor for a plurality of pieces of habit data, and then compare the mean value with diagnostic data. In addition, the diagnostic apparatus may compare habit data with diagnostic data, and then calculate a mean value among the comparison results.

In operation 303, the diagnostic apparatus determines whether the user is at risk of a disease based on the comparison result obtained in operation 301. Specifically, in response to a determination made in operation 301 that the sum is greater than a preset threshold, the diagnostic apparatus determines that the user is at risk of the corresponding disease. At this point, the preset threshold may be changed.

In response to a determination made in operation 303 that the user is at risk of the disease, the diagnostic apparatus may provide preventive measures according to a predetermined procedure. For example, the diagnostic apparatus may display information on the disease or inform a doctor or a family member of a result of the determination. In addition, the diagnostic apparatus may provide the user's habit data together.

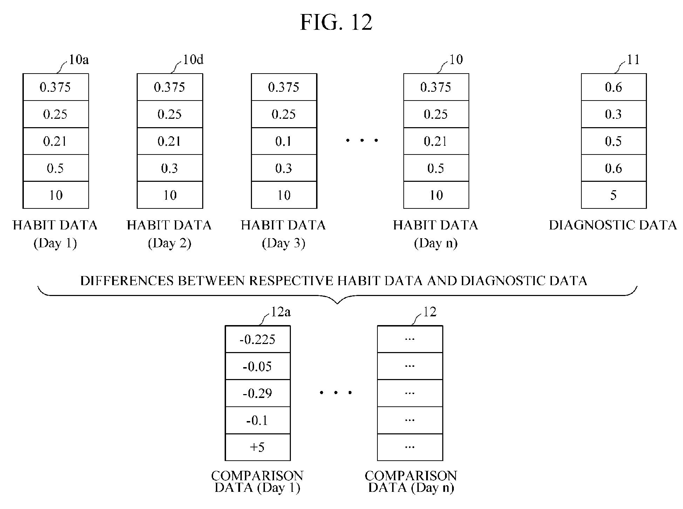

FIG. 12 is a diagram for explaining an example of operation 103 shown in FIG. 9.

Referring to FIGS. 11 and 12, in operation 301, a diagnostic apparatus may generates comparison data 12 indicating calculated differences between respective habit factors for habit data 10 and corresponding habit factors for diagnostic data 11. Then, in operation 303, the diagnostic apparatus may determine whether the user is at risk of a disease by comparing a sum of the calculated differences with a threshold.

Meanwhile, the method illustrated in FIGS. 11 and 12 are described with reference to a diagnostic apparatus, but the same method may be used for a diagnostic management apparatus to perform diagnosis of a disease.

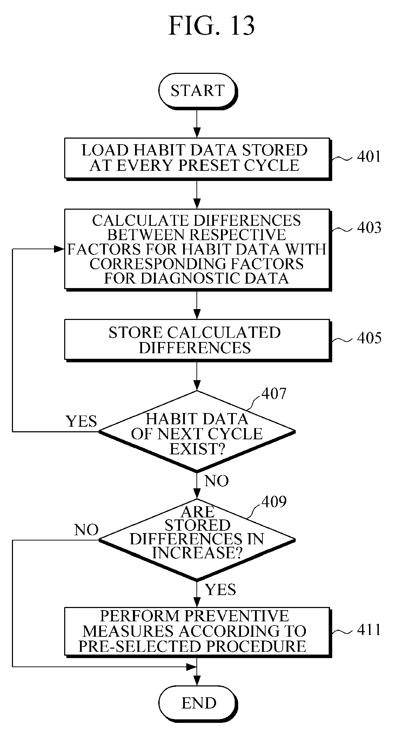

FIG. 13 is a flow chart illustrating another example of operation 103 shown in FIG. 9.

Referring to FIG. 13, in operation 401, a diagnostic apparatus loads habit data that are stored at every preset cycle. For example, the diagnostic apparatus may load all the habit data that are periodically generated once a day. Meanwhile, the preset cycle may be changed according to setting information.

The diagnostic apparatus calculates differences between respective habit factor for a single piece of habit data and corresponding habit factors for diagnostic data in operation 403, and stores the differences in operation 405. In the case where a piece of habit data includes one or more habit factors, the diagnostic apparatus generates comparison data by calculating differences between respective habit factors for the habit data and corresponding habit factors for the diagnostic data, and stores the generated comparison data.

In operation 407, the diagnostic apparatus determines whether there is habit data generated at the next cycle. In the case where there is habit data generated at the next cycle, the diagnostic apparatus goes back to operation 403 to calculate a differential value between the habit data generated at the next cycle and the diagnostic data.

Alternatively, in the case where there is no habit data generated at the next cycle, the diagnostic apparatus moves on into operation 409 to determine whether the stored differential value has a tendency to increase. At this point, the diagnostic apparatus may determine that the differential value has a tendency to increase, only when the increase is greater than a preset threshold. For example, in the case where a threshold is set as an increase by 0.3 for three days, the diagnostic apparatus may determine a stored differential value has a tendency to increase, if the stored differential value has increased by 0.9 (1.11 -> 1.30 -> 2.01).

In response to a determination in operation 409 that the differential value has a tendency to increase, the diagnostic apparatus may take preventive measures according to a predetermined procedure in operation 411.

That is, the diagnostic apparatus calculates a plurality of comparison results, and, in operation 303, determines whether a user is at risk of a disease by comparing a mean of the comparison results with a threshold.

The method illustrated in FIG. 13 is described with reference to a diagnostic apparatus, but the same method may be used for a diagnostic management apparatus to perform diagnosis of a disease.

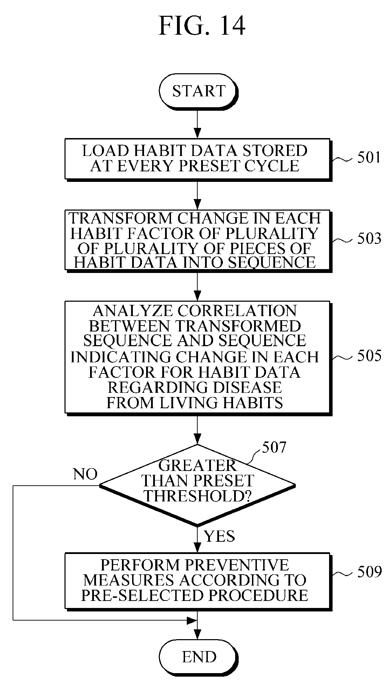

FIG. 14 is a flow chart illustrating still another example of operation 103 shown in FIG. 9.

FIG. 15 is a diagram illustrating yet another example of operation 103 shown in FIG. 9.

Referring to FIGS. 14 and 15, in operation 501, a diagnostic apparatus loads habit data that are stored at every preset cycle. For example, the diagnostic apparatus thirty pieces of habit data that are generated once a day.

In operation 503, the diagnostic apparatus transform a change in the loaded habit data into a sequence 21. For example, the diagnostic apparatus may transform a change in thirty pieces of habit data which are generated every day, into the sequence 21 as shown in FIG. 15.

In operation 505, the diagnostic apparatus calculates a correlation coefficient by analyzing the sequence 21 and a sequence 22 that indicates a change in each habit factor for habit data of a patient suffering from a disease. At this point, the diagnostic apparatus may generate an unknown sequence value by performing regression analysis.

In operation 507, the diagnostic apparatus determines whether the correlation coefficient 23 is greater than a preset threshold. For example, in the case where a threshold is 0.8 and a correlation coefficient is 0.826, the diagnostic apparatus may determine that the correlation coefficient is greater than the threshold.

In response to a determination made in operation 507 that the correlation coefficient is greater than the threshold, the diagnostic apparatus may provide preventive measures according to a preset procedure.

The method illustrated in FIGS. 14 and 15 is described with reference to a diagnostic apparatus, but the same method may be used for a diagnostic management apparatus to perform diagnosis of a disease.

Those who are skilled in the related art may understand that various and specific modifications may be made without modifying the technical ideas or essential characteristics of the invention. Accordingly, the embodiments disclosed above are exemplary, and should be understandable not to be limited to in all aspects.

## Claims

1. A diagnostic apparatus based on habits, comprising:
a habit analyzer configured to generate a user's habit data by analyzing the user's behavior data, such as sensor data, detected from a diagnostic apparatus, such as at least one sensor; and
a diagnoser configured to determine whether the user is at risk of a disease, by comparing the generated habit data with diagnostic data, wherein the diagnostic data is habit data of healthy people.

2. The diagnostic apparatus of claim 1, wherein the habit analyzer is further configured to comprise a log analyzing module to generate the habit data by analyzing log data stored in a usage log.

3. The diagnostic apparatus of claim 1 or 2, wherein the habit analyzer is further configured to comprise an input analyzing module to generate the habit data by analyzing data input by the user.

4. The apparatus diagnostic of claim 1, 2 or 3, wherein the habit analyzer is further configured to generate the habit data in a normalized form.

5. The diagnostic apparatus of any one or more than one of the preceding claims, wherein the diagnoser is further configured to comprise a search module to search the diagnostic data that matches profile information of the user.

6. The apparatus diagnostic of any one or more than one of the preceding claims, wherein the diagnoser is further configured to, in response to a differential value between the habit data and the diagnostic data being greater than a preset threshold, determine that the user is at risk of the disease.

7. The diagnostic apparatus of any one or more than one of the preceding claims, further comprising:
a storage configured to store the habit data that are generated at every preset cycle,
wherein the diagnoser is further configured to comprise either or both of:
- a tendency analyzing module to determine whether a differential value between the stored habit data and the diagnostic data has a tendency to increase, and, in response to determining that the differential value has a tendency to increase, determine that the user is at risk of the disease; and
- a correlation analyzing module to transform a change in a user's habit data stored in the storage into a sequence and analyze correlation between the transformed sequence and a sequence indicating a change in habit data of a patient suffering from a specific disease, and to, in response to the correlation being greater than a preset threshold, determine that the user is at risk of the specific disease.

8. The diagnostic apparatus of any one or more than one of the preceding claims, further comprising:
a preventive measure provider configured to, in response to a determination that the user is at risk of the disease, either provide the user with information on the disease or inform a doctor or a family member of a result of the determination.

9. The diagnostic apparatus of any one or more than one of the preceding claims, wherein the behavior data received from the diagnostic apparatus comprises at least one of sensor data detected from one or more sensors of the diagnostic apparatus, data directly input by the user through the diagnostic apparatus, and log data stored in a usage log of the diagnostic apparatus.

10. A diagnostic method based on habits, comprising:
generating a user's habit data by analyzing the user's behavior data, such as sensor data, detected from a diagnostic apparatus, such as at least one sensor;
searching for diagnostic data that match with profile information of a user, wherein the diagnostic data are habit data of healthy people; and
determining whether the user is at risk of a disease, by comparing the user's habit data with the found diagnostic data.

11. The diagnostic method of claim 10, further comprising:
generating the habit data based on the user's behavior data;
wherein the behavior data comprises at least one of sensor data detected from one or more sensors, data directly input by the user, and log data stored in a usage log.

12. The diagnostic method of claim 10 or 11, wherein the generating of the habit data is normalizing the habit data.

13. The diagnostic method of claim 10, 11 or 12, wherein the determining of whether the user is at risk of the disease comprises, in response to a differential value between the habit data and the diagnostic data being greater than a preset threshold, determining that the user is at risk of the disease.

14. The diagnostic method of any one or more than one of the preceding claims 10 - 13, wherein the generating of the habit data comprises storing the habit data generated at every preset cycle, and
determining of whether the user is at risk of the disease by either or both of:
- determining whether a differential value between the stored habit data and the diagnostic data has a tendency to increase, and, in response to a determination that the differential value has a tendency to increase, determining that the user is at risk of the disease, and
- transforming a change in the stored habit data into a sequence, analyzing correlation between the transformed sequence with a sequence that indicates a change in habit data of a patient suffering from a disease, and, in response to the correlation being greater than a preset threshold, determining that the user is at risk of the disease.

15. The diagnostic method of any one or more than one of preceding claims 10 - 14, further comprising:
in response to a determination that the user is at risk of the disease, either providing the user with information on the disease or informing a doctor or a family member of a result of the determination.
